# EUROPEAN PATENT SPECIFICATION

(11) **EP 1 987 041 B1**
(45) Date of publication and mention of the grant of the patent: **22.05.2013**
(21) Application number: 07712302.4
(22) Date of filing: 23.02.2007
(51) Int. Cl.: C07D 501/00, C12P 35/06, C12N 15/81, C12R 1/645

(54) **IMPROVED CEPHALOSPORIN PRODUCTION**
VERBESSERTE HERSTELLUNG VON CEPHALOSPORIN
AMÉLIORATION DE LA PRODUCTION DE CÉPHALOSPORINE

(30) Priority: 23.02.2006 EP 06110332
(43) Date of publication of application: 05.11.2008
(73) Proprietor: DSM Sinochem Pharmaceuticals Netherlands B.V., 2613 AX Delft (NL)
(72) Inventor: HANS, Marcus, NL-2582 BN Den Haag (NL); BOVENBERG, Roelof, Ary, Lans, NL-3062 DA Rotterdam (NL); VAN DEN BERG, Marco Alexander, NL-2685 EH Poeldijk (NL); DRIESSEN, Arnold Jacob Mattieu, NL-9727 DA Groningen (NL); NIJLAND, Jeroen Gerben, NL-9531 JG Borger (NL)
(74) Representative: Misset, Onno
(86) International application number: PCT/EP2007/051755
(87) International publication number: WO 2007/096419

(56) References cited:
- EP-B1- 0 532 341
- EP-B1- 0 540 210
- WO-A-2004/106347
- ULLAN R V ET AL: "The cefT gene of Acremonium chrysogenum C10 encodes a putative multidrug efflux pump protein that significantly increases cephalosporin C production" MGG MOLECULAR GENETICS AND GENOMICS, vol. 267, no. 5, July 2002 (2002-07), pages 673-683, XP002427397 ISSN: 1617-4615
- MARTIN J F ET AL: "Secretion systems for secondary metabolites: how producer cells send out messages of intercellular communication" CURRENT OPINION IN MICROBIOLOGY, CURRENT BIOLOGY LTD, GB, vol. 8, no. 3, June 2005 (2005-06), pages 282-293, XP004936711 ISSN: 1369-5274
- LIRAS PALOMA ET AL: "Gene clusters for beta-lactam antibiotics and control of their expression: why have clusters evolved, and from where did they originate?" INTERNATIONAL MICROBIOLOGY, vol. 9, no. 1, March 2006 (2006-03), pages 9-19 URL, XP002427398 ISSN: 1139-6709
- GARDINER D M ET AL: "The ABC transporter gene in the sirodesmin biosynthetic gene cluster of Leptosphaeria maculans is not essential for sirodesmin production but facilitates self-protection" FUNGAL GENETICS AND BIOLOGY, SAN DIEGO, CA, US, vol. 42, no. 3, March 2005 (2005-03), pages 257-263, XP004737355 ISSN: 1087-1845

## Description

The present invention relates to the fermentative production of a cephalosporin in a microorganism.

The filamentous fungus *Penicillium chrysogenum* is a natural producer of β-lactam antibiotics, such as penicillin-G. In the last decades, penicillin-G production capabilities were highly improved by classical strain improvement, resulting in powerful industrial production strains. Recently, by means of metabolic pathway engineering, several routes for cephalosporin production were successfully integrated in penicillin production strains, in particular *Penicillium chrysogenum.* In EP-A-0532341, *Penicillium chrysogenum* has been transformed with the *Streptomyces clavuligerus cefE* gene encoding an expandase which enables the transformed strain to produce adipyl-7-ADCA (adipyl-7-amino-3-methyl-3-cephem-4-carboxylic acid) when cultured in the presence of the precursor adipic acid. In EP-A-0540210, in addition to the *Streptomyces clavuligerus cefE* gene, *Penicillium chrysogenum* has been transformed with a hydroxylase gene whose expression product converts the 3-methyl side chain of adipyl-7-ADCA to 3-hydroxymethyl, to give adipyl-7-aminodeacetylcephalosporanic acid (adipyl-7-ADAC); in another example in EP-A-0540210 *Penicillium chrysogenum,* already transformed with genes encoding expandase and hydroxylase, is further transformed with an acetyltransferase gene whose expression product converts the 3-hydroxymethyl side chain to the 3-acetyloxymethyl side chain to give adipyl-7-ACA. In WO2004/106347, a strain of *Penicillium chrysogenum* has been transformed with genes encoding an expandase, a hydroxylase and an O-carbamoyl transferase enzyme resulting in adipyl-7-amino-3-carbamoyloxymethyl-3-cephem-4-carboxylic acid.

Industrial strains of *Penicillium chrysogenum* have been selected for their capacity to produce and secrete large quantities of a variety of beta-lactam compounds, in particular penicillin G and penicillin V, into the culture medium. Various lines of evidence indicate that beta-lactam secretion is an active process, possibly via transport proteins. During the latter stages of such fermentation processes extremely high beta-lactam titers are found in fermentations on an industrial scale. WO01/32904 discloses several ABC-transporter polypeptides that are involved in the secretion of penicillin G. Enhancing an ABC transporter activity resulted in an enhanced secretion of the beta-lactam compound.

The production of cephalosporin molecules in naturally non-cephalosporin recombinant production hosts such as the *Penicillium* strains discussed hereinbefore still imposes several problems. For example, the secretion level of the desired compounds is still relatively low and must be increased in order to be both technically and economically more attractive. The production rate of cephalosporins produced in *P. chrysogenum* will be limited due to the fact that the penicillin transporters, i.e. the ABC transporter polypeptides, will have a low affinity for the cephalosporin compound. It has been suggested in WO01/32904 that this could be overcome by cloning one or more homologous ABC transporter polypeptides, from natural cephalosporin producers such as *Acremonium chrysogenum* using the polynucleotides encoding the ABC-transporters disclosed in WO01/32904.

The filamentous fungus *Acremonium chrysogenum* is a natural producer of cephalosporins such as cephalosporin C. In a particular strain of this fungus, *Acremonium chrysogenum* C10, a CefT gene has been identified recently which encodes a putative efflux pump protein and, at the same time, significantly increases cephalosporin C production (Ullán et al. (2002) Mol. Genet. Genomics, 267, 673-683; Liras and Martin (2006) International Microbiology 9, 9-19; Martin et al. (2005) Curr. Opinion Microbiol. 8, 282-293). The nucleotide sequence of the CetT-gene has been deposited in the nucleotide database of the National Center for Biotechnology Information under accession number (locus) AJ487683. The NCBS can be accessed on the INTERNET at www.ncbi.nlm.nih.gov and the CefT gene nucleotide sequence at http://www.ncbi.nlm.nih.gov/entrez/viewer.fcgi?db=nucleotide&val=21214008. From the amino acid sequence of the protein encoded by the CefT, the authors concluded that it is a transmembrane protein since it has 12 transmembrane segments (TMS) and contains motifs A, B, C, D2 and G characteristic of the Drug:H+ antiporter 12 TMS group of the major facilitator superfamily. The CefT protein confers resistance to some toxic organic acids, including isovaleric acid and phenylacetic acid, as was concluded from the fact that these acids were toxic to an *Acremonium* strain in which the CefT was made inactive. Overexpression of the homologous CefT gene resulted in an improved cephalosporin C production in *Acremonium chrysogenum.* This was not the case when a truncated form of the CefT gene was used. However, targeted inactivation of the CefT gene did not affect the cephalosporin C production in *Acremonium chrysogenum* which suggests that there are redundant systems involved in cephalosporin export in *Acremonium chrysogenum.*

The CefT protein from *Acremonium chrysogenum* C10 does not belong to the family of ABC-transporters. Comparison of the amino acid sequence of the CefT protein from *Acremonium chrysogenum* C10 (Ullán et al (2002)) with any of the amino acid sequence of the ABC-transporters disclosed in WO01/32904 reveals that the CefT protein does not have any homology with the ABC transporter proteins (i.e. <25%).

The term "CefT gene" relates to a gene encoding a CefT protein, i.e. a protein involved in the transport of a cephalosporin compound from inside the cephalosporin producing microorganism towards the outside of the cephalosporin producing microorganism. An example of such a CefT gene from the strain *Acrymonium chrysogenum* C10 can be found in Ullán et al (2002) which also discloses the amino acid sequence of the CefT protein encoded by the CefT-gene.

The term 'heterologous CefT gene' means that the CefT gene may be obtained from any suitable host microorganism and is used to transform a cephalosporin-producing microorganism, with the proviso that the suitable host microorganism from which the CefT gene is derived is not the same species as the cephalosporin-producing microorganism transformed with the CefT gene.

The term 'homologous CefT gene" means that CefT gene is obtained from and any suitable host microorganism and used to transform a cephalosporin-producing microorganism, proviso that the suitable host microorganism from which the CefT gene is derived is the same species as the cephalosporin-producing microorganism transformed with the CefT gene. Transformation of the strain *Acrymonium chrysogenum* C10 with a homologous CefT gene originating from the same strain *Acrymonium chrysogenum* C10 has been described by Ullán et al. (2002).

The term 'homology' refers to the identity of the sequence of a first gene, protein or enzyme with the sequence of a second gene, protein or enzyme. Throughout this description, the degree of homology is expressed as a percentage of identity with a reference gene, protein or enzyme. Determining these homologies can be performed by methods known to the skilled man, for instance by using the protein sequence of the reference protein as a "query sequence" to perform a search against public databases to, for example, identify other family members or related sequences. Such searches can be performed using BLAST programs (version 2.2) using the default parameters of the respective program. See for instance http://www.ncbi.nlm.nih.gov

In a first aspect, the present invention provides a cephalosporin-producing microorganism characterized in that it has been transformed with a heterologous *CefT* gene and whereby the transformed microorganism has an improved cephalosporin-producing capacity compared with the microorganism not being transformed with the heterologous CefT gene. An improved cephalosporin-producing capacity is defined herein as the ratio of the cephalosporin production capacity of the transformed microorganism compared to the cephalosporin production capacity of the microorganism not being transformed with the heterologous CefT gene being at least 1.2, more preferably at least 1.4, more preferably at least 1.6, more preferably at least 1.8, more preferably at least 2.0, more preferably at least 2.1, more preferably at least 2.3, more preferably at least 2.5, more preferably at least 3.0, more preferably at least 5.0, more preferably at least 10. The cephalosporin-producing microorganism is preferably a microorganism that is by nature capable of producing beta-lactam compounds such as penicillins and cephalosporins and may be selected from the group consisting of *Penicillium, Aspergillus, Streptomyces* and *Acremonium.* In this group, several microorganisms possess the capacity to produce cephalosporins by nature such as *Streptomyces* and *Acremenium or* penicillins by nature such as *Penicillium* and *Aspergillus.* Other microorganisms, such as *Penicillium* as disclosed hereinbefore, may have acquired the capacity to produce cephalosporins by genetic engineering such as *Penicillium chrysogenum* which, after having being transformed with a gene encoding expandase, is capable of producing N-acylated derivatives of 7-ADCA or, when further being transformed with hydroxylase, a hydroxylase and an acetyltransferase or with hydroxylase and an acetyltransferase and a O-carbamoyl transferase is capable of producing N-acylated derivatives 7-ADAC, 7-ACA and 7-amino-3-carbamoyloxymethyl-3-cephem-4-carboxylic acid respectively.

Preferably the heterologous *CefT* gene may be derived from any suitable cephalosporin producing microorganism, but is preferably derived from *Acremonium,* more preferably from *Acremonium chrysogenum,* most preferably from *Acremonium chrysogenum* C10 as disclosed by Ullán et al (2002). For the purpose of the invention, also a homologue of a suitable heterologous *CefT* gene may be used, for instance a CefT gene encoding a CefT protein with more than 30% homology, preferably more than 40% homology, preferably more than 50% homology, preferably more than 60% homology, preferably more than 70% homology, preferably more than 80% homology, preferably more than 90% homology on protein basis when compared to the CefT protein from *Acremonium chrysogenum* C10 as disclosed by Ullán et al (2002).

A preferred embodiment of the present invention is a *Penicillium* strain, preferably *Penicillium chrysogenum,* that has acquired the capacity to produce an N-substituted 7-ADCA derivative by genetic engineering as a result of its transformation with a gene encoding an expandase - for example as disclosed in EP-A-0532341 - and in addition has been transformed with a heterologous CefT gene, preferably with a CefT gene from *Acremonium,* more preferably with a CefT gene from *Acremonium chrysogenum;* most preferred is the CefT gene from *Acremonium chrysogenum* C10 encoding the CefT protein with the amino acid sequence as has been disclosed in Ullán et al (2002). Another preferred embodiment of the present invention is a *Penicillium* strain, preferably *Penicillium chrysogenum,* that has acquired the capacity to produce a N-acylated 7-ADAC derivative, by genetic engineering as a result of its transformation with a gene encoding an expandase and a hydroxylase as disclosed in EP-A-0540210 and in addition has been transformed with a heterologous CefT gene, preferably with a CefT gene from *Acremonium,* more preferably with a CefT gene from *Acremonium chrysogenum.;* most preferred is the CefT gene from *Acremonium chrysogenum* C10 encoding the CefT protein with the amino acid sequence as has been disclosed in Ullán et al (2002). Another preferred embodiment of the present invention is a *Penicillium* strain, preferably *Penicillium chrysogenum,* that has acquired the capacity to produce a N-acylated 7-ACA derivative, by genetic engineering as a result of its transformation with a gene encoding an expandase and a hydroxylase and a acyltransferase as disclosed in EP-A-0540210 and in addition has been transformed with a heterologous CefT gene, preferably with a CefT gene from *Acremonium,* more preferably with a CefT gene from *Acremonium chrysogenum;* most preferred is the CefT gene from *Acremonium chrysogenum* C10 encoding the CefT protein with the amino acid sequence as has been disclosed in Ullán et al (2002). Another preferred embodiment of the present invention is *a Penicillium* strain, preferably *Penicillium chrysogenum,* that has acquired the capacity to produce a N-acylated 7-amino-3-carbamoyloxymethyl-3-cephem-4-carboxylic acid derivative, by genetic engineering as a result of its transformation with a gene encoding an expandase, a hydroxylase and an O-carbamoyl transferase as disclosed in W02004/106347and in addition has been transformed with a heterologous CefT gene, preferably with a CefT gene from *Acremonium,* more preferably with a CefT gene from *Acremonium chrysogenum;* most preferred is the CefT gene from *Acremonium chrysogenum* C10 encoding the CefT protein with the amino acid sequence as has been disclosed in Ullán et al (2002).

In a second aspect, the present invention provides a method for the construction of the cephalosporin-producing microorganism according to the invention which comprises the steps of isolating the heterologous *CefT* gene from a suitable host microorganism and transforming the cephalosporin-producing microorganism with the isolated CefT gene with the proviso that the host microorganism is not the same species as the cephalosporin-producing microorganism. In a preferred embodiment, the present invention provides a method for the construction of the cephalosporin-producing microorganism according to the invention which comprises the steps of isolating the heterologous *CefT gene* from *Acremonium,* preferably *Acremonium chrysogenum,* most preferably *Acremonium chrysogenum* C10 as described by Ullán et al (2002) and transforming the cephalosporin-producing microorganism, preferably *Penicillium,* more preferably *Penicillium chrysogenum* transformed with a gene encoding an expandase and optionally one or more of the genes encoding a hydroxylase, acyltransferase and O-carbamoyl transferase thus providing the *Penicillium* strain with the capacity to produce 7-ADCA, 7-ADAC, 7-ACA and N-acylated 7-amino-3-carbamoyloxymethyl-3-cephem-4-carboxylic acid as described before, with the isolated CefT gene.

In a third aspect the invention provides a process for the production of a cephalosporin comprising culturing the corresponding cephalosporin-producing microorganism of the invention according to methods known in the art. Preferred embodiments of the invention are processes for the production of N-acylated derivatives of 7-ADCA, 7-ACA, 7-ADCA and 7-amino-3-carbamoyloxymethyl-3-cephem-4-carboxylic acid. A preferred embodiment of the invention is a process for the production of the adipyl derivate of 7-ADCA, 7-ACA, 7-ADCA or 7-amino-3-carbamoyloxymethyl-3-cephem-4-carboxylic acid using fermentation processes as disclosed in EP-A-0532341, EP-A-0540210 and WO2004/106347.

### EXAMPLES

### General materials and methods

Standard procedures were carried out as described in Sambrook, J. et al. (1989), Molecular cloning: a laboratory manual, 2nd Ed., Cold Spring Harbor Laboratory Press, Cold Spring Harbor, New York. DNA was amplified using the proofreading polymerases Turbo-Pfu-Polymerase or Herculase (Stratagene, The Netherlands), following the manufacturers protocol, while the verification of constructed strains and plasmids was achieved by using Taq polymerase. Restriction enzymes were from Invitrogen or New England Biolabs. For routine cloning, *Escherichia coli* strains Top10 and DH10B (Invitrogen) were employed. Verification of the constructed plasmids was carried out by restriction analysis and subsequent sequencing (Seqlab GmbH, Goettingen, Germany).

The filamentous fungus *Penicillium chrysogenum* Wisconsin 54-1255 (ATCC 28089) was used as a host for genetic modification and production strain construction.

### Example 1

### Construction of the CefT expression cassette for transformation in Penicillium chrysogenum

*Escherichia coli* DH5a was used as host strain for high frequency transformation, plasmid DNA amplification (Sambrook, 1989) and plasmid construction using the Multisite Gateway^{®} Three-Fragment Vector Construction Kit of Invitrogen with different vectors. Vector pDONR™ P4-P1R was used for cloning of the promotor region of 920bp upstream of the pcbC gene. The attB4F site was added by Polymerase Chain Reaction (PCR) using primer attB4-F-IPNS and the attB1R site was added with primer attB1-R-IPNS. The amplified PCR product was cloned into pDONR™ P4-P1R using BP clonase creating pDONR™ P4-P1R-IPNS promotor. Vector pDONR™ 201 gateway vector was used to clone the *CefT* gene of *Acremonium chrysogenum* (acCefT). The attB1F site was added by PCR using primer attB1F-AcCefT and the attB2R site was added using attB2R-AcCefT-minus-Stop. The amplified AcCefT gene was cloned into pDONR™ 201 using BP clonase yielding pDONR™ 201-AcCefT. The stop codon of the AcCefT was removed to enable fusions with a His-tag or GFP-tag at the carboxyl terminal site of the gene. To add the attB2F site to the terminator region of 551 bp downstream of the penDE gene for cloning in the pDONR™ P2R-P3 primer attB2F-His8x-Tat was used. Together with primer attB3R-Tat the terminator region was cloned and a HIS-tag with a stop codon was added to create vector pDONR™ P2-P3R-HIS-Tat. To obtain a carboxyl-terminal fusion with GFP primers attB2R-GFP and attB3R-Tat were used to create vector pDONR™ P2-P3R-GFP-Tat.

Combining these pDONR™ vectors in the pDEST™ R4-R3 in the LR reaction of the Multisite Gateway^{®} Three-Fragment Vector Construction Kit a destination vector was made with respectively the PcbC promotor, AcCefT gene, His- or GFP-tag and the terminator of the PenDE gene.

**Table 1. Primers used for Gateway^{®} cloning. Nucleotides in bold represent the att recombination sites of the Gateway^{®} system, in italic represent nucleotides to maintain the frame between the HIS-tag, the GFP-tag and AcCefT gene and underlined nucleotides represent the 8 amino acids of the HIS-tag.**

| | |
|---|---|
| attB4-F-IPNS | GGGGACAACTTTGTATAGAAAAGTTGTCCTTATACTGGGCCTGCTG CATTGG |
| attB1-R-IPNS | GGGGACTGCTTTTTTGTACAAACTTGCGTCTAGAAAAATAATGGTGA AAACTTGAAGGCGT |
| attB1F-AcCefT | GGGGACAAGTTTGTACAAAAAAGCAGGCTCTATGGCGAACAATTCT GGAACAACACACAGTG |
| attB2R-AcCefT-minus-Stop | GGGGACCACTTTGTACAAGAAAGCTGGGTCAGGGTTGCTCACGTAG CTGATAGCC |
| attB2F-His8x-Tat | GGGGACAGCTTTCTTGTACAAAGTGGTCCACCATCACCATCACCAT CACCATTGAAGGCTCTTCATGACGAGCCAATGC |
| attB2F-GFP | GGGGACAGCTTTCTTGTACAAAGTGGTCATGGTGAGCAAGGGCGAG GAG |
| attB3R-Tat | GGGGACAACTTTGTATAATAAAGTTGCCTGAAAGAGTTGATATTGAA GGTAGTAGG |

### Example 2

### Transformation of Penicillium chrysogenum ATCC 28089 CefE/CefF/CmcH (construction described in WO2004106347) with the CefT expression cassette designed in Example 1

For the transformation experiments of *Penicillium chrysogenum* ATCC 28089 *CefE*/*CefF*/*CmcH* a strain was used that produced circa 0.3 g/L adipyl-7-amino-3-carbamoyloxymethyl-3-cephem-4-carboxylic acid. Protoplasts of were made and isolated and transformation (Alvarez, 1987) was done by co-transformation of the pDEST™ P4-R3 vector with pcbC promotor, *CefT* gene and penDE terminator and the AMDS-gene used as selection marker on plates with acetamide as sole Nitrogen source. Total RNA of transformants was isolated using Trizol^{®} (Invitrogen) and positive transformants were determined with RT-PCR beads (Amersham) using a AcCefT specific primer (AcCefT F1; 5'-TCGATTCGTACCAGCACCAGGC-3') and a attB2 - HIS-tag primer (5'-TGGTGATGGTGATGGTGGACCACTT-3') to obtain a PCR fragment of 384bp.

### Example 3

### Cultivation of Penicillium chrysogenum ATCC 28089 CefE/CefF/CmcH CefT and measurement of adipyl-7-amino-3-carbamoyloxymethyl-3-cephem-4-carboxylic acid secretion

Mycelium of 5 CefT transformants was inoculated in a 50 mL shake flask in mineral medium containing (g/L): glucose (5); lactose (80); urea (4.5); (NH₄)₂SO₄ (1.1); Na₂SO₄ (2.9); KH₂PO₄ (5.2); K₂HPO₄ (4.8) and 10 mUL of a trace element solution A containing citric acid (150); FeSO₄.7H₂O (15); MgSO₄.7H₂O (150); H₃BO₃ (0.0075); CuSO₄.5H₂O (0.24); CoSO₄.7H₂O (0.375); ZnSO₄.7H₂O (5); MnSO₄.H₂O (2.28); CaCl₂.2H₂O (0.99); 3 g/L adipic acid; pH before sterilization 6.5. After 7 days growth at 25°C, 280 rpm, cells were pelleted and the supernatant was analyzed using NMR for the formation of cephalosporins. See table 2 for the results. The data clearly show the positive effect of CefT gene integration in the genomes of *P. chrysogenum* on cephalosporin production.

**Table 2: Concentration of adipyl-7-amino-3-carbamoyloxymethyl-3-cephem-4-carboxylic acid in shake flask cultures of the CefT gene transformants of Penicillium chrysogenum ATCC 28089 CefE/CefF/CmcH.**

| Strain | Concentration of adipyl-7-amino-3-carbamoyloxymethyl-3-cephem-4-carboxylic acid | Ratio |
|---|---|---|
| Strain | 0.30 g/L | 1.0 |
| Strain CefT1 | 0.55 g/L | 1.8 |
| Strain CefT2 | 0.49 g/L | 1.6 |
| Strain CefT3 | 0.63 g/L | 2.1 |
| Strain CefT4 | 0.71 g/L | 2.4 |
| Strain CefT5 | 0.53 g/L | 1.8 |

## Claims

1. A cephalosporin-producing *Penicillium* strain which has acquired the capacity of producing cephalosporins after having being transformed with a gene encoding expandase, and optionally, further being transformed with a gene encoding hydroxylase, a hydroxylase and an acetyltransferase or with hydroxylase and an acetyltransferase and a O-carbamoyl transferase said Penicillium being capable of producing N-acylated derivatives 7-ADCA, 7-ADAC, 7-ACA and 7-amino-3-carbamoyl-oxymethyl-3-cephem-4-carboxylic acid respectively **characterized in that** it has been transformed with a heterologous *CefT* gene encoding a CefT protein which is a protein involved in the transport of a cephalosporin compound from inside the cephalosporin producing microorganism towards the outside of the cephalosporin producing microorganism and whereby the ratio of the cephalosporin production capacity of the *Penicillium* strain transformed with the heterologous CefT gene compared to the cephalosporin production capacity of the *Penicillium* strain not being transformed with the heterologous CefT gene is at least 1.2.

2. A cephalosporin-producing microorganism according to claim 1 **characterized in that** the heterologous *CefT* gene encoding a CefT protein which is a protein involved in the transport of a cephalosporin compound from inside the cephalosporin producing microorganism towards the outside of the cephalosporin producing microorganism is derived from *Acremonium,* preferably from *Acremonium chrysogenum,* most preferably from *Acremonium chrysogenum* C10 or is a homologue of said CefT gene encoding a CefT protein with more than 30% identity on protein basis when compared to the CefT protein from *Acremonium chrysogenum* C10.

3. A method for the construction of a *Penicillium* strain according to anyone of claims 1-2 comprising the steps of:
a. Isolating the CefT gene from a suitable host; and
b. Transforming the cephalosporin-producing *Penicillium* strain with the CefT gene isolated in step a.
with the proviso that the host and the cephalosporin-producing microorganism are not the same organism.

4. A process for the production of a cephalosporin comprising culturing the cephalosporin-producing *Penicillium* strain according to anyone of claim 1-2.

5. A process according to claim 4 wherein the cephalosporin is selected from the group consisting of the N-acylated, derivatives of 7-ADCA, 7-ACA, 7-ACCA and 7-amino-3-carbamoyl-oxymethyl-3-cephem-4-carboxylic acid.

## Patentansprüche

1. Cephalosporin produzierender *Penicillium*-Stamm, der die Fähigkeit zur Produktion von Cephalosporinen nach seiner Transformation mit einem für Expandase codierenden Gen und gegebenenfalls weiteren Transformation mit einem für Hydroxylase codierenden Gen, einer Hydroxylase und einer Acetyltransferase oder mit Hydroxylase und einer Acetyltransferase und einer O-Carbamoyl-Transferase erworben hat, wobei das Penicillium in der Lage ist, N-acylierte Derivate 7-ADCA, 7-ADAC, 7-ACA bzw. 7-Amino-3-carbamoyloxymethyl-3-cephem-4-carbonsäure zu produzieren, **dadurch gekennzeichnet, dass** er mit einem heterologen CefT-Gen, das für ein CefT-Protein codiert, bei dem es sich um ein am Transport einer Cephalosporinverbindung aus dem Inneren des Cephalosporin produzierenden Mikroorganismus nach außerhalb des Cephalosporin produzierenden Mikroorganismus beteiligtes Protein handelt, transformiert wurde, und wobei das Verhältnis der Cephalosporinproduktionskapazität des mit dem heterologen CefT-Gen transformierten *Penicillium*-Stamms verglichen mit der Cephalosporinproduktionskapazität des nicht mit dem heterologen CefT-Gen transformierten *Penicillium-*Stamms wenigstens 1,2 beträgt.

2. Cephalosporin produzierender Mikroorganismus nach Anspruch 1, **dadurch gekennzeichnet, dass** das heterologe CefT-Gen, das für ein CefT-Protein codiert, bei dem es sich um ein am Transport einer Cephalosporinverbindung aus dem Inneren des Cephalosporin produzierenden Mikroorganismus nach außerhalb des Cephalosporin produzierenden Mikroorganismus beteiligtes Protein handelt, aus *Acremonium,* vorzugsweise aus *Acremonium chrysogenum,* am stärksten bevorzugt aus *Acremonium chrysogenum* C10 stammt oder es sich dabei um ein Homolog des CefT-Gens handelt, das für ein CefT-Protein mit mehr als 30% Identität auf Proteinbasis verglichen mit dem CefT-Protein aus *Acremonium chrysogenum* C10 codiert.

3. Methode zur Konstruktion eines *Penicillium*-Stamms nach einem der Ansprüche 1-2, umfassend die Schritte:
a. Isolieren des CefT-Gens aus einem geeigneten Wirt; und
b. Transformieren des Cephalosporin produzierenden *Penicillium*-Stamms mit dem in Schritt a. isolierten CefT-Gen,
mit der Maßgabe, dass es sich bei dem Wirt und dem Cephalosporin produzierenden Mikroorganismus nicht um den gleichen Organismus handelt.

4. Verfahren zur Herstellung eines Cephalosporins, bei dem man den Cephalosporin produzierenden Penicillium-Stamm nach einem der Ansprüche 1-2 kultiviert.

5. Verfahren nach Anspruch 4, wobei das Cephalosporin aus der aus den N-acylierten Derivaten von 7-ADCA, 7-ACA, 7-ACCA und 7-Amino-3-carbamoyloxymethyl-3-cephem-4-carbonsäure bestehenden Gruppe ausgewählt ist.

## Revendications

1. Souche de *Penicillium* productrice de céphalosporines qui a acquis la capacité de produire des céphalosporines après avoir été transformée par un gène codant pour l'expandase, et éventuellement après en outre avoir été transformée par un gène codant pour l'hydroxylase, une hydroxylase et une acétyltransférase ou par l'hydroxylase et une acétyltransférase et une 0-carbamoyltransférase, ledit *Penicillium* étant capable de produire des dérivés N-acylés 7-ADCA, 7-ADAC, 7-ACA et acide 7-amino-3-carbamoyl-oxyméthyl-3-céphem-4-carboxylique respectivement, **caractérisée en ce qu'**elle a été transformée par un gène *CefT* hétérologue codant pour une protéine CefT qui est une protéine impliquée dans le transport d'un composé céphalosporine de l'intérieur du microorganisme producteur de céphalosporines vers l'extérieur du microorganisme producteur de céphalosporines et le rapport de la capacité de production de céphalosporines de la souche de *Penicillium* transformée par le gène CefT hétérologue en comparaison à la capacité de production de céphalosporines de la souche de *Penicillium* non transformée par le gène CefT hétérologue étant d'au moins 1,2.

2. Microorganisme producteur de céphalosporines selon la revendication 1, **caractérisé en ce que** le gène *CefT* hétérologue codant pour une protéine CefT qui est une protéine impliquée dans le transport d'un composé céphalosporine de l'intérieur du microorganisme producteur de céphalosporines vers l'extérieur du microorganisme producteur de céphalosporines est issu de *Acremonium,* de préférence de *Acremonium chrysogenum,* de façon tout à fait préférable de *Acremonium chrysogenum* C10 ou est un homologue dudit gène *CefT* codant pour une protéine CefT ayant plus de 30 % d'identité sur la base des protéines en comparaison à la protéine CefT provenant de *Acremonium chrysogenum* C10.

3. Méthode de construction d'une souche de *Penicillium* selon l'une ou l'autre des revendications 1 et 2, comprenant les étapes consistant à :
a. isoler le gène *CefT* d'un hôte convenable ; et
b. transformer la souche de *Penicillium* productrice de céphalosporines par le gène CefT isolé à l'étape a.,
à condition que l'hôte et le microorganisme producteur de céphalosporines ne soit pas le même organisme.

4. Procédé de production d'une céphalosporine comprenant la culture de la souche de *Penicillium* productrice de céphalosporines selon l'une ou l'autre des revendications 1 et 2.

5. Procédé selon la revendication 4, **caractérisé en ce que** la céphalosporine est choisie dans le groupe constitué par les dérivés N-acylés de 7-ADCA, de 7-ACA, de 7-ACCA et d'acide 7-amino-3-carbamoyl-oxyméthyl-3-céphem-4-carboxylique.
